Europäisches Patentamt

European Patent Office

Office européen des brevets

(19)

(11) **EP 0 763 500 A2**

(12) **EUROPÄISCHE PATENTANMELDUNG**

(43) Veröffentlichungstag:
   **19.03.1997 Patentblatt 1997/12**

(51) Int. Cl.$^6$: **C01B 21/24**, B01D 53/04,
   B01J 20/26, A61K 33/00

(21) Anmeldenummer: 96114414.4

(22) Anmeldetag: **09.09.1996**

(84) Benannte Vertragsstaaten:
   **AT DE ES FR GB IT SE**

(30) Priorität: **18.09.1995 DE 19534248**

(71) Anmelder: **HOECHST AKTIENGESELLSCHAFT
   65926 Frankfurt am Main (DE)**

(72) Erfinder:
   • **Sixl, Wolfgang, DPh.
     65929 Frankfurt (DE)**
   • **Schleicher, Andreas, Dr.
     65614 Beselich (DE)**
   • **Frank, Georg, Dr.
     72074 Tübingen (DE)**
   • **Vaahs, Tilo, Dr.
     65510 Idstein (DE)**

(54) **Verfahren zur Herstellung von NO-2-freiem Stickstoffmonoxid**

(57)    Das Verfahren zur Herstellung von $NO_2$-freien, NO-haltigen Gasen basiert auf der Verwendung eines schwefelhaltigen Polymers in Gegenwart von Wasser als Filtermaterial. Ein schwefelhaltiges Polymer ist beispielsweise Polyphenylensulfid.

Es wird ein Filter zur Entfernung von $NO_2$ beschrieben, das ein schwefelhaltiges Polymer und Wasser enthält. Der Filter wird zum Beispiel zur Erzeugung von $NO2$ - freiem $NO/N_2/O_2$-Gasgemischen für medizinische Applikationen verwendet.

EP 0 763 500 A2

Printed by Rank Xerox (UK) Business Services
2.13.17/3.4

**Beschreibung**

Die Erfindung betrifft ein Filtermaterial und ein Verfahren zur Entfernung von Stickstoffdioxid ($NO_2$) aus Gasen oder Flüssigkeiten mittels schwefelhaltiger Polymere in Gegenwart von Wasser.

DE 43 28 450 A1 beschreibt ein Filtermaterial und ein Verfahren zur Entfernung von Stickoxiden aus Gasen und Flüssigkeiten. Polyarylenthioether werden als polymeres Filtermaterial für Stickstoffdioxid eingesetzt. In einer Reaktion mit einem Polyarylenthioether wird Stickstoffdioxid zu Stickstoffmonoxid umgesetzt. Das Filtermaterial ist auch für die Entfernung von Stickstoffmonoxid geeignet, wenn dem Filtermaterial ein Oxidationsmittel mit einem Redoxpotential von mindestens 0,96 V gegen Standard-Wasserstoffelektrode (SHE) beigemischt wird.

$NO_2$-freies NO-Gas oder NO-Gasgemische werden in der Abgasmeßtechnik zur Kalibrierung von Meß- und Analysesystemen benötigt.

In jüngster Zeit hat der medizinische Einsatz von Stickstoffmonoxid (NO) besondere Bedeutung erlangt. Bei Patienten mit schweren pulmonalen Krankheitsbildern kann durch Zudosierung von NO zur Atemluft der Bluthochdruck im Lungenkreislauf gesenkt werden. Verbunden mit der bronchodilatorischen Wirkung von NO kommt es zu einer verbesserten Belüftung diverser Lungenabschnitte und damit auch zu einem verbesserten Gasaustausch. Das zum Einsatz kommende NO-haltige Gas darf nach Möglichkeit kein $NO_2$ wegen dessen Toxizität enthalten.

Es wurde gefunden, daß bei der Entfernung von $NO_2$ aus Gasen oder Flüssigkeiten mittels schwefelhaltiger Polymere als Filtermaterial die Filterkapzität von schwefelhaltigen Polymeren in Gegenwart von Wasser bzw. Wasserdampf, z.B. Leuftfeuchtigkeit, deutlich verbessert wird.

Die Erfindung betrifft ein Verfahren zur Entfernung von $NO_2$ aus Gasen oder Flüssigkeiten, dadurch gekennzeichnet, daß das Gas oder Flüssigkeit in Gegenwart von Wasser mit einem Material in Kontakt gebracht wird, das ein schwefelhaltiges Polymer enthält.

Schwefelhaltige Polymere sind Polymere, die Struktureinheiten mit einer Schwefelbrücke (-S-) enthalten. Bevorzugt sind als schwefelhaltige Polymere, Polymere, die mindestens eine Arylensulfid-Einheit enthalten, beispielsweise Polyarylensulfide. Besonders bevorzugt sind Polymere mit mindestens einer Phenylensulfid-Einheit ($-C_6H_4-S-$), beispielsweise Polyphenylensulfid.

Die Entfernung von $NO_2$ aus Gasen oder Flüssigkeiten mittels Polyarylensulfiden oder schwefelhaltigen Polymeren sind in DE 43 28 450 A1 und der deutschen Patenanmeldung P 44 19 860.4 (Anmeldetag: 7.Juni 1994) mit dem Titel: "Verfahren und Filter zur Herstellung von $NO_2$-freiem Stickstoffmonoxid mit schwefelhaltigen Polymeren", beschrieben, worauf Bezug genommen wird.

Schwefelhaltige Polymere wie Polyarylenthioether zeigen gegenüber NO eine vernachlässigbar geringe Wechselwirkung. Daher ist es mit Hilfe schwefelhaltiger Polymere möglich, selektiv $NO_2$ neben NO zu entfernen Dieser Effekt kann beispielsweise zur Herstellung von $NO_2$-freiem Stickstoffmonoxid oder $NO_2$-freien NO-haltigen Gasen oder Flüssigkeiten genutzt werden.

Ein Filtermaterial, das ein schwefelhaltiges Polymer enthält, bietet die Möglichkeit ein sehr leistungsfähiges Filter für $NO_2$ herzustellen. Enthält das Filtermaterial zusätzlich ein geeignetes Oxidationsmittel, so kann ein Filter für NO und $NO_2$ hergestellt werden.

Der Begriff "Filtermaterial" steht für ein schwefelhaltiges Polymer oder für ein schwefelhaltiges Polymer-enthaltendes Filtermaterial. Ein schwefelhaltiges Polymerenthaltendes Filtermaterial kann beispielsweise auch ein Polymer-Blend sein. Das Filtermaterial kann verschiedenste Formen haben wie Pulver, Faser, Stapelfaser, Langfaser (continuous fiber), Gewebe, Vlies, Sinterstoffe oder Formkörper. Poröse Strukturen und große Oberflächen sind vorteilhaft.

Das Wasser kann in flüssiger oder gasförmiger Form dem zu reinigenden Gas, der zu reinigenden Flüssigkeit oder dem Filtermaterial zugegeben werden. Die Wasserzugabe kann vor oder während des Filterprozesses erfolgen.

Es gibt viele Möglichkeiten, wodurch eine Gegenwart von Wasser bei der Reaktion von $NO_2$ mit einem schwefelhaltigen Polymer erreicht werden kann:

- Verwendung von befeuchtetem Gas oder wasserhaltiger Flüssigkeit,
- Einsatz von befeuchtetem Filtermaterial (Befeuchtung zum Beispiel durch Behandlung mit Wasserdampf),
- Einsatz von wasserenthaltendem Filtermaterial (zum Beispiel herstellungsbedingter Wassergehalt, in Poren enthaltenes Wasser, im Bulk des Materials eingeschlossenes Wasser),
- Zusatzstoffe im Filtermaterial, die Wasser freisetzen können (zum Beispiel Kristallwasser-haltige Verbindungen, so wird bei der Reduktion von Natriumperborat Kristallwasser freigesetzt).

Bei der Filterung von $NO_2$ aus feuchten oder befeuchteten Gasen ist es günstig, wenn der Wassergehalt des Gases so bemessen ist, daß eine Kondensation von Wasser auf dem Filtermaterial vermieden wird, da die Bildung eines Wasserfilmes auf dem Filtermaterial den Transport von $NO_2$ zum Filtermaterial behindert. Zur optimalen Wassermenge im Gasraum gilt: je höher die Wasserkonzentration um so besser, solange es nicht zur Kondensation auf dem Filtermaterial kommt. Dabei ist die optimale Wassermenge natürlich von der Temperatur abhängig. Günstig ist zum Beispiel eine 90% relative Gasfeuchtigkeit.

Günstig für eine Entfernung von $NO_2$ in Gegenwart von Feuchtigkeit sind auch Zusätze im Filtermaterial, die eine Adsorption oder Konzentration von Wassermolekülen an dem Filtermaterial begünstigen. Solche Zusätze sind beispielsweise polare Verbindungen, hydrophile Verbindungen, hygroskopische Verbindungen. Die Zusätze können anorganische Verbindungen wie Salze, Mineralien (z.B. Silikate, Kieselgel, Glas, mineralische Füllstoffe) oder organische Verbindungen wie hydrophile Polymere, Verbindungen mit polaren Gruppen.

Eine permanente Zuführung von Wasser über den zu reinigenden Gas- bzw. Flüssigkeitsstrom zum Filtermaterial, z.B. durch erhöhte Gasfeuchtigkeit, ist günstig. Das Verfahren gemäß der Erfindung kann bei jeder Temperatur, die unterhalb des Erweichungspunktes der verwendeten Polymere liegt, durchgeführt werden. Im allgemeinen liegen die Anwendungtemperaturen im Bereich von minus 30 bis +240°C, vorzugsweise minus 10 bis +200°C, besonders bevorzugt von 10 bis 80°C. Für Anwendungen im medizinischen Bereich eignet sich die Körpertemperatur, z.B. 37°C. Im allgemeinen wird die Filterleistung mit steigender Temperatur verbessert.

Die erforderliche Kontaktzeit des zu reinigenden Mediums mit dem Filtermaterial ist u.a. abhängig von der Strömungsgeschwindigkeit, der Verweilzeit, der Oberfläche des Filtermaterials, der Geometrie des Filters, der Temperatur. Im allgemeinen liegt die Kontaktzeit im Bereich von 0.001 Sekunden bis 10 Minuten, vorzugsweise 2 Sekunden bis 5 Minuten.

Das Verfahren gemäß der Erfindung ist anwendbar z.B. bei Gasen mit einem NO-Gehalt zwischen 60 Vol.-% und 1 ppb, vorzugsweise 50 Vol.-% und 10 ppb und besonders bevorzugt zwischen 40 Vol.-% und 50 ppb. Der abtrennbare $NO_2$-Gehalt liegt zwischen 50 % und 1 ppb, vorzugsweise 20 Vol.-% und 10 ppb und besonders bevorzugt zwischen 10 Vol.-% und 10 ppb. Das Verhältnis zwischen NO und $NO_2$ in den zu behandelnden Gasen oder Flüssigkeiten kann dabei zwischen 1000000 : 1 und 1 : 1000000, vorzugsweise zwischen 10000 : 1 und 1 : 10000 und besonders bevorzugt zwischen 1000 : 1 und 1 : 1000 liegen.

Weiterer Gegenstand der Erfindung ist ein Filtermaterial oder Filter zur Entfernung von $NO_2$ aus Gasen oder Flüssigkeiten, dadurch gekennzeichnet, daß es ein schwefelhaltiges Polymer und Wasser enthält.

Das Filtermaterial oder Filter kann auch in Kombination mit anderen Filtermaterialien, z.B. Staubfiltern, verwendet werden.

Das Filter kann das Filtermaterial z. B. in Form von einem Pulver-Schüttbett, einem Vlies, einer Vlies-Pulver-Mischung, einer Gitter- oder Wabenstruktur enthalten. Das Pulver kann aber auch in Vliese aus anderen Werkstoffen eingearbeitet werden. Das Verfahren und das Filter sind besonders geeignet für die Herstellung von NO-Prüfgasen, wobei z.B. ein NO-Rohgas, das mit $NO_2$ verunreinigt ist und eine hohe NO-Konzentration enthält, durch das Filter geleitet wird und anschließend auf die gewünschte Konzentration mit einem sauerstofffreien Gas verdünnt wird. Die Schritte der Verdünnung und der Filterung können aber auch parallel oder in umgekehrter Reihenfolge durchgeführt werden.

Weiter können das Verfahren und das Filter gemäß der Erfindung Anwendung in der Medizintechnik finden. So kann beispielsweise bei einer Behandlung mit einer NO-Aufnahme über die Lunge das NO-haltige Gas und die zugesetzte Luft vor oder im Filter vereinigt und damit erreicht werden, daß ein $NO_2$-freies Gasgemisch eingeatmet wird. Das Filter kann zum Beispiel aus einer Atemmaske bestehen, in deren Zuluftstrom das schwefelhaltige Polymer enthaltene Filter eingesetzt ist.

Das Filter gemäß der Erfindung kann zur Erzeugung von $NO_2$-freien Stickstoffmonoxid-Stickstoff-Luft-Gemischen für die Behandlung von IRDS (IRDS = Infant Respiratory Distress Syndrome), ARDS ( = Acute Respiratory Distress Syndrome, auch = Adult Respiratory Distress Syndrome), Lungenversagen, Migräne, persistierende pulmonale Hypertonie basierend auf Linksherzinsuffizienz oder zur Verbesserung der Lungenfunktion eingesetzt werden.

Weiter können das Verfahren und das Filter z.B. Anwendung in der Medizintechnik finden. Bei einer Behandlung mit einer NO-Aufnahme über die Lunge kann beispielsweise das NO-haltige Gas und die zugesetzte Luft vor dem Filter vereinigt und befeuchtet werden und dann durch das Filter geleitet werden. Ein feuchtes $NO_2$-freies Gasgemisch kann dann eingeatmet werden.

Bei der Behandlung der Lunge mit NO-haltigen Gasen erweist sich die Verwendung des Filters in Kombination mit einem sogenannten Respirator als sehr vorteilhaft. Das Filter kann unter anderem eingesetzt werden a) in der Gaszuführung unmittelbar am Patienten, b) als Zwischenfilter nach der Mischstufe NO/Luft/Sauerstoff und c) als Sicherheitsfilter nachgeschaltet zur NO-Quelle (zur Sicherheit bei einem Sauerstoff-Einbruch in die Versorgungsleitung - Gefahr der $NO_2$-Bildung).

Das Filter kann z.B. aus einer Atemmaske bestehen, in deren Zuluftstrom das schwefelhaltige Polymer enthaltene Filtermaterial eingesetzt ist, wobei das Filtermaterial wasserhaltig oder befeuchtet ist oder für eine Befeuchtung des Atemgases vor der Filterung gesorgt wird.


Beispiele:

1.) Ein Gasgemisch von 900 ppm $NO_2$ in synthetischer Luft (Messer Griesheim GmbH, Sondergaswerk, 47009 Duisburg, Deutschland) wurde in einem Gasmisch- und Dosiersystem, bestehend aus Flow-Controllern (Typ 1259C) und dem zugehörigen Kontrollgerät (Typ 247C, beide von MKS Instruments, 81829 München, Bundesrepublik Deutschland) mit einem Gasgemisch von 900 ppm NO in $N_2$ so gemischt, daß ein Gasgemisch mit 855 ppm

$NO_2$ und 45 ppm NO in einer 19:1 Mischung der beiden Trägergase synthetische Luft und $N_2$ entstand. Danach wurde dieser Gasstrom in zwei Teilströme aufgeteilt, ein Teilstrom ging zur Befeuchtung zu einer mit Wasser gefüllten Waschflasche und wurde danach mit dem anderen unveränderten Teilstrom wieder zusammengeführt. Durch Veränderung der Verhältnisse der beiden Teilströme konnten verschiedene Feuchtigkeitsgrade eingestellt werden, die nach dem Zusammenführen der beiden Zweige gemessen werden konnten (Feuchtigkeitsmesser Modell 610 von Testo GmbH & Co., 79846 Lenzkirch, Deutschland). Das befeuchtete Gas wurde über eine Filterpatrone geleitet, die mit Poly-p-phenylensulfid (PPS) in Granulatform (mittlerer Teilchendurchmesser ca. 1 mm) gefüllt war. Die Absorptionsstrecke in der Filterpatrone ist durch folgende Parameter gekennzeichnet:

| Innendurchmesser: | 3 cm |
|---|---|
| Masse: | 15 g |
| Schütthöhe: | 6 cm |
| Durchsatz: | 100 l/h |
| Strömungsgeschwindigkeit: | 4 cm/s |
| Temperatur: | 25 °C |

Das Gas wurde nach Durchgang durch die Filterpatrone zur Analyse des NO und $NO_2$ Gehalts in ein NO/$NO_2$-Chemolumineszensmeßgerät (Typ CLD 700 El Ht, Eco Physics AG, Durnten, Schweiz; minimale Nachweisgrenze 0.1 ppm, Linearität +/- 1 % vom Vollausschlag), Meßbereichseinstellung 0-1000 ppm, geleitet.

Die Filterwirkung auf $NO_2$ tritt sofort ein. Es wurde aber keine Filterwirkung bezüglich NO festgestellt.

Nachfolgende Ergebnisse zeigen die Vergrößerung der Filterkapazität des Filtermaterials mit steigender Gasfeuchte. Die Filterwirkung bleibt dabei nahezu konstant.

Die Filterkapazität K wird hier definiert als die Masse des aufgenommenen $NO_2$ bezogen auf die Filtermaterialmasse in Prozent, entweder gerechnet bis zum Zeitpunkt, bei dem nach dem Filter die $NO_2$-Konzentration des Gasstroms mehr als 10 % der Eingangskonzentration beträgt ($K_{10\%}$), oder gerechnet bis zu einem bestimmten Zeitpunkt t=x min ($K_{xmin}$) .

Die $NO_2$-Filterwirkung ist definiert als der Prozentsatz der $NO_2$-Eingangskonzentration der gefiltert wurde.

Ergebnis:

| Luftfeuchte: | max. $NO_2$-Filterwirkung: | Filterkapazität $K_{10\%}$: |
|---|---|---|
| 0,2% | >97 % | 0,2 % |
| 32% | >92 % | 0,25% |
| 66% | >91 % | 0,3% |
| 99% | >90 % | 0,5% |
| kondensiert | >90 % | 0,5 % |

2.) In einem weiteren Versuch, wurde wie in Beispiel 1.) 855 ppm $NO_2$ und 45 ppm NO über die Filterpatrone geleitet. Im Unterschied zu Beispiel 1 wurde einmal das gesamte Gas über die Waschflasche geleitet und einmal das trockene Gas über die Filterpatrone geleitet, die jeweils mit einem Polyarylenthioether (PPS, aus IR-Messung ergab es sich als Poly-para[2,6-phenylensulfid]) in Stapelfaserform (Durchmesser ca. 10 μm) gefüllt war.

| Innendurchmesser: | 3 cm |
|---|---|
| Masse: | 3 g |
| Schütthöhe: | 15 cm |
| Durchsatz: | 50 l/h |
| Strömungsgeschwindigkeit: | 2 cm/s |
| Temperatur: | 25° C |

Ergebnis:
Die Filterwirkung auf $NO_2$ tritt sofort ein. Es wurde aber keine Filterwirkung bezüglich NO festgestellt.

| Luftfeuchte: | >99% | 0 % |
|---|---|---|
| max. $NO_2$-Filterwirkung: | >50 % | >50 % |
| Filterkapazität ($K_{30min}$): | 1,4 % | 0,1 % |

3.) In einem weiteren Versuch, wurde wie in Beispiel 1.) 855 ppm $NO_2$ und die 45 ppm NO über die Filterpatrone geleitet. Im Unterschied zu Beispiel 1 wurde einmal das gesamte Gas über die Waschflasche geleitet und die Filterpatrone war mit einem befeuchteten Polyarylenthioether (PPS, aus IR-Messung ergab es sich als Polypara[2,6-phenylensulfid]) in "continuous fiber" Form (Durchmesser ca. 10 $\mu$m) gefüllt. In einem zweiten Versuch war das Gasgemisch und das PPS trocken.

| Innendurchmesser: | 4 cm |
|---|---|
| Masse: | 10 g |
| Schütthöhe: | 5 cm |
| Durchsatz: | 50 l/h |
| Strömungsgeschwindigkeit: | 1,1 cm/s |
| Temperatur: | 25° C |

Ergebnis:
Die Filterwirkung auf $NO_2$ tritt sofort ein. Es wurde aber keine Filterwirkung bezüglich NO festgestellt.

| Luftfeuchte: | >99% (Wasser auf PPS kondensiert) | 0 % |
|---|---|---|
| $NO_2$-Filterung: | >86 % | >50 % |
| Filterkapazität ($K_{150min}$): | 4 % | 0,1 % |

**Patentansprüche**

1. Verfahren zur Entfernung von $NO_2$ aus Gasen oder Flüssigkeiten, dadurch gekennzeichnet, daß das Gas oder die Flüssigkeit in Gegenwart von Wasser mit einem Material in Kontakt gebracht wird, das ein schwefelhaltiges Polymer enthält.

2. Verfahren nach Anspruch 1, dadurch gekennzeichnet, daß das schwefelhaltige Polymer ein Polyarylenthioether ist.

3. Verfahren nach Anspruch 1 oder 2, dadurch gekennzeichnet, daß das schwefelhaltige Polymer Polyphenylensulfid ist.

4. Verfahren nach einem oder mehreren der Ansprüche 1 bis 3 zur Erzeugung von $NO_2$-freiem NO-Gas oder $NO_2$-freien NO-Gasgemischen.

5. Verfahren nach einem oder mehreren der Ansprüche 1 bis 4, dadurch gekennzeichnet, daß Wasser in Form von Luftfeuchtigkeit zugesetzt wird.

6. Verfahren nach einem oder mehreren der Ansprüche 1 bis 5, dadurch gekennzeichnet, daß das schwefelhaltige Polymer Wasser enthält.

7. Verfahren nach einem oder mehreren der Ansprüche 1 bis 6, dadurch gekennzeichnet, daß Wasser aus im schwefelhaltigen Polymer enthaltenen Zusatzstoffen freigesetzt wird.

8. Filtermaterial zur Entfernung von $NO_2$ aus Gasen oder Flüssigkeiten, dadurch gekennzeichnet, daß es ein schwefelhaltiges Polymer und Wasser enthält.

9. Filter zur Entfernung von $NO_2$ aus Gasen oder Flüssigkeiten, dadurch gekennzeichnet, daß es ein schwefelhaltiges Polymer und Wasser enthält.

10. Verwendung eines Filters nach Anspruch 9 als Atemschutzfilter.

11. Verwendung eines Filters nach Anspruch 9 zur Erzeugung von $NO_2$-freien Prüfgasen.

12. Verwendung eines Filters nach Anspruch 9 zur Erzeugung von $NO_2$-freien NO/Stickstoff/Luft-Gemischen für medizinische Applikationen, in denen das NO über die Lunge aufgenommen wird.